# EUROPEAN PATENT APPLICATION

(11) **EP 2 241 278 A1**
(43) Date of publication of application: **20.10.2010**
(21) Application number: 10250769.6
(22) Date of filing: 14.04.2010
(51) Int. Cl.: A61B 17/34

(54) **Cannula with sealing elements**

(30) Priority: 15.04.2009 US 424213
(71) Applicant: Ethicon Endo-Surgery, Inc., Cincinnati, OH 45242 (US)
(72) Inventor: Shelton, Frederick E., IV, Hillsboro, Ohio 45133 (US); Widenhouse, Christopher W., Clarksville, Ohio 45113 (US)
(74) Representative: Tunstall, Christopher Stephen

(57) **Abstract**

Methods and devices are provided for providing surgical access into a body cavity. In one embodiment, a surgical access device is provided that includes an elongate hollow tube having a proximal end that is configured to be positioned adjacent to a tissue surface and a distal end configured to extend through a body wall into a body cavity. The proximal end of the device can be can be configured to allow the proximal-most portion of the surgical access device to rest flush or substantially flush against a tissue surface of a patient when the device is inserted into the patient. In some embodiments, the elongate hollow tube can have a non-circular cross-section.

## Description

### FIELD OF THE INVENTION

The present invention relates to methods and devices for performing surgical procedures, and in particular to methods and devices for accessing a body cavity.

### BACKGROUND OF THE INVENTION

Abdominal laparoscopic surgery gained popularity in the late 1980s, when benefits of laparoscopic removal of the gallbladder over traditional (open) operation became evident. Reduced postoperative recovery time, markedly decreased post-operative pain and wound infection, and improved cosmetic outcome are well established benefits of laparoscopic surgery, derived mainly from the ability of laparoscopic surgeons to perform an operation utilizing smaller incisions of the body cavity wall.

Laparoscopic procedures generally involve insufflation of the abdominal cavity with CO₂ gas to a pressure of around 15 mm Hg. The abdominal wall is pierced and a 5-10 mm in diameter straight tubular cannula or trocar sleeve is then inserted into the abdominal cavity. A laparoscopic telescope connected to an operating room monitor is used to visualize the operative field, and is placed through a the trocar sleeve. Laparoscopic instruments (graspers, dissectors, scissors, retractors, etc.) are placed through two or more additional trocar sleeves for the manipulations by the surgeon and surgical assistant(s).

Recently, so-called "mini-laparoscopy" has been introduced utilizing 2-3 mm diameter straight trocar sleeves and laparoscopic instruments. When successful, mini-laparoscopy allows further reduction of abdominal wall trauma and improved cosmesis. Instruments used for mini-laparoscopic procedures are, however, generally more expensive and fragile. Because of their performance limitations, due to their smaller diameter (weak suction-irrigation system, poor durability, decreased video quality), mini-laparoscopic instruments can generally be used only on selected patients with favorable anatomy (thin cavity wall, few adhesions, minimal inflammation, etc.).

Trocars, including those used in so-called "mini-laparoscopy," typically include seals within a housing at their proximal end to prevent the escape of insufflation gases. The size of the housing that contains the seals can limit the ability to place multiple instruments in close proximity. A proximal seal housing also increases the profile of the trocar, can limit the range of motion of the trocar, and can constrain the movement of instruments inserted through the trocar.

Thus, there is a need for improved methods and devices for laparoscopic surgery with a reduced profile that allow increased range of motion of instruments inserted through the trocar

### SUMMARY OF THE INVENTION

The present invention generally provides methods and devices for providing surgical access into a body cavity. In one exemplary embodiment, a surgical access device is provided that includes a elongate hollow tube having a proximal end that is configured to be positioned adjacent to a tissue surface and a distal end configured to extend through a body wall into a body cavity.

The device can have various configurations. In one embodiment, the proximal end can include a rim with a distal surface that contacts a tissue surface and a proximal surface that forms the proximal-most end of the tube and defines an opening extending into a working channel of the hollow tube. In some embodiments, the rim can gradually extend radially outward from the longitudinal axis of the elongate hollow tube. In addition to defining an opening extending into a working channel of the tube, the radially expanding proximal-most end can be configured to rest against a tissue surface when a distal portion of the elongate hollow tube is disposed through an opening in tissue and into a body cavity, thus providing a low-profile trocar that sits flush or just proud of the tissue surface.

The shape and materials of the elongate hollow tube can vary in any number of ways. In one exemplary embodiment, the elongate hollow tube can have a non-circular cross-section. The non-circular cross-section can be any shape such as a polygon. For example, the non-circular cross-section can be triangular. In another embodiment, the elongate hollow tube can be rigid.

The surgical access device can include at least one sealing element configured to seal the working channel when no instrument is inserted therethrough and/or to form a seal around an instrument inserted through the sealing element. The sealing element can have any number of variations and can be disposed at any point within the elongate hollow tube. For example, the sealing element can be spaced a distance apart from the proximal-most end or the distal-most end of the elongate hollow tube. In one exemplary embodiment, the sealing element includes first and second sealing elements. For example, the sealing element can comprise a first sealing element, and the device can include a second sealing element disposed within the hollow tube and configured to seal the working channel of the elongate hollow tube when no instrument is disposed through the second sealing element. In another embodiment, the first sealing element can be a unitary molded structure held within a groove formed in an inner surface of the elongate tube. In other embodiments, the sealing element and the elongate hollow tube can be formed from different materials.

Methods for accessing a body cavity are also provided, and in one embodiment the method can include inserting a distal portion of a cannula through an opening formed in tissue to position the distal portion of the cannula within a body cavity. Inserting the cannula can also include positioning a proximal-most portion of the cannula against a tissue surface and positioning at least one sealing element disposed within the cannula within at least one of the opening in the tissue and the body cavity. The method can further include inserting an instrument through an opening in the proximal-most end of the cannula and through a working channel of the cannula to position a distal end of the instrument in the body cavity. In an exemplary embodiment, the at least one sealing element can form a seal around the instrument.

The cannula can be configured in any number of ways. For example, in some embodiments the proximal-most portion of the cannula extends radially outward to engage the tissue surface and to prevent the proximal-most end of the cannula from passing through the opening in the tissue. In other embodiments, a second sealing element can be disposed within the cannula. The second sealing element can be positioned within at least one of the opening in the tissue and the body cavity. In some embodiments, the second sealing element forms a seal in the working channel when no instrument is inserted therethrough.

### BRIEF DESCRIPTION OF THE DRAWINGS

The invention will be more fully understood from the following detailed description taken in conjunction with the accompanying drawings, in which:

FIG. 1 is a perspective view of a surgical access device;

FIG. 2A is a cross-sectional view of the surgical access device of FIG. 1 showing the circular cross-sectional shape of the device;

FIG 2B is a cross-sectional view of another embodiment of a surgical access device having a triangular cross-sectional shape;

FIG. 2C is a cross-sectional view of another embodiment of surgical access device of having a square cross-sectional shape;

FIG. 3A is a cut-away perspective view of the surgical access device of FIG. 1 showing the sealing elements;

FIG. 3B is a cut-away perspective view of another embodiment of a surgical access device having multiple sealing elements;

FIG. 4 is a schematic view of the surgical access device of FIG. 1 showing an instrument disposed therethrough and positioned at various angles;

FIG. 5 is a perspective view of one embodiment of a duckbill sealing element for use in a surgical access device; and

FIG. 6 is an exploded view of one embodiment of an instrument seal.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention generally provides improved surgical access devices. In use, the surgical access devices disclosed herein can provide access through tissue into to a patient's body cavity. The access device, or cannula, can be positionable within an opening in a patient's body such that a distal portion of the access device extends into a patient's body cavity and a proximal portion rests adjacent to the patient's skin on an exterior of the patient's body. A lumen in the access device can form a pathway or working channel through the opening in a patient's body so that surgical instruments can be inserted from outside the body to an interior body cavity. In certain exemplary embodiments, the improved surgical access device eliminates the need for a housing at the proximal end of the device. The elimination of the typical housing reduces the profile of the proximal end of the device, thus maximizing the range of motion of the cannula as well as reducing constriction points between the cannula and inserted instruments and also improving the ability to place multiple devices in close proximity to each other. However, while no housing is preferred, a person skilled in the art will appreciate that the use of a housing is not outside the scope of the present invention. For example, the access device can include a low profile annular structure that provides support to the device.

A person skilled in the art will appreciate that the access device can be placed in any opening within a patient's body, whether a natural orifice or an opening made by an incision. For example, the access device can be placed through the umbilicus, endoscopically including, vaginally, percutaneously, etc. The elasticity of the skin of the patient can assist in the retention of the access device in the body opening or incision made in the body. In one embodiment, the access device can be substantially flexible so that it can easily be maneuvered into and within tissue as needed. In such an embodiment, the access device can include a rigid housing or support structure. The housing or support can be configured to support multiple access devices as well. In other embodiments, the access device can be rigid or semi-rigid. The access device can be formed of any suitable material known in the art, for example silicone, urethane, thermoplastic elastomer, and rubber. In other aspects, the durometer of the materials used to form the access device can vary in different portions of the device. For example, a portion of the device positioned within tissue can be more rigid than a portion positioned in a body cavity.

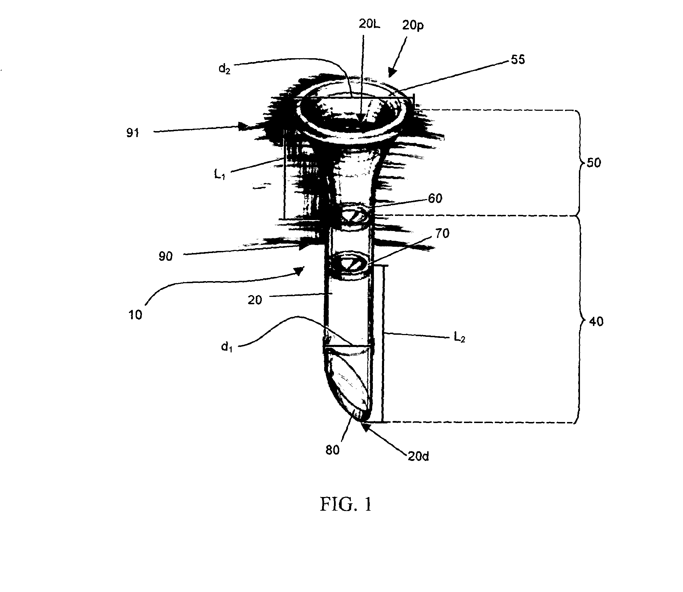

FIG. 1 illustrates one exemplary embodiment of a surgical access device, or cannula, 10 that generally includes an elongate hollow tube 20 with a proximal end 20p and a distal end 20d. The elongate hollow tube 20 defines a lumen 20L through which instruments can be inserted from outside the body to an interior body cavity. In one exemplary embodiment, the nominal outer diameter d₁ of the elongate hollow tube 20 can be between approximately 2 mm to 3 mm. In some embodiments, the proximal end 20p of the elongate hollow tube 20 can include a rim 55 with a diameter d₂ that is larger than the nominal diameter d₁ of the elongate hollow tube 20. In the embodiment shown in FIG. 1, a proximal region 50 of the elongate hollow tube 20 includes a region in which the diameter of the elongate hollow tube 20 varies, for example increases from the nominal diameter d₁ to a maximum diameter d₂ at the rim 55. The maximum diameter d₂ can be defined as a multiple of the nominal diameter d₁. For example, the maximum diameter d₂ can be between 1.5 to 2 times the nominal diameter d₁. A distal region 40, i.e., the remainder of the tube extending distally from the proximal region 50, can have a fixed diameter, i.e., the nominal diameter of the hollow tube 20. However, in other embodiments the diameters of the proximal and distal regions can have various other configurations.

The proximal region 50 of the elongate hollow tube can have various shapes. For example, in the illustrated embodiment, the diameter of the elongate hollow tube 20 changes smoothly, e.g., tapers, from the nominal diameter d₁ to a maximum diameter d₂, forming a proximal region 50 that has a flared shape and ends in a rim 55. In other embodiments, the proximal region 50 can be substantially conical or can have an exponentially changing diameter, such as, for example, a substantially hyperbolic cone. In other embodiments, the proximal region can be in the form of a flange or lip extending radially outward from a constant diameter tube.

The proximal or inner surface of the proximal region 50 defines an opening at the proximal end 20p of the elongate hollow tube 20 through which an instrument can be inserted. As will be discussed in more detail below, the proximal surface of the flared proximal region 50 can have any number of shapes. For example, the flared proximal region 50 can provide a smooth surface that can guide an instrument into the working channel as it is inserted.

The distal or outer surface of the proximal region 50 can also have any number of shapes. For example, the distal surface of the proximal region 50 can be configured to allow the proximal-most portion of the surgical access device to rest flush or substantially flush against a tissue surface 90 of a patient when the cannula 10 is inserted into the patient. A rim 55 defines the transition between the proximal or inner surface of the proximal region 50 and the outer or distal surface of the proximal region 50. A person having ordinary skill in the art will appreciate that the rim can be formed in any shape. For example, the rim can be a cylindrical ring formed at the proximal-most end of the elongate hollow tube 20. The rim 55 can also be designed to engage the tissue surface 90 to hold the cannula 10 in the desired position. For example, the shape and diameter of the proximal portion, including the maximum diameter d₂ at the rim 55, can be configured to prevent the rim 55 from passing through the opening formed in the tissue surface 90. In another example, the shape and diameter of the flared proximal portion, including the maximum diameter d₂ at the rim 55, can engage an opening having a smaller diameter to produce a force sufficient to resist the forces applied to instruments inserted through the elongate hollow tube 20.

The length of the elongate hollow tube 20 between the proximal end 20p and the distal end 20d can vary. For example, the length can be minimized to provide maximum range of motion for instruments inserted through the cannula 10 as discussed in more detail below. For example, the overall length of the cannula can be 75 mm, 100 mm, or 150 mm. In other exemplary embodiments, the overall length of the cannula can be between 75 mm and 150 mm.

The length of the proximal region 50 can also vary. For purposes of the following examples, the proximal region can be defined as the region in which the diameter of the elongate hollow tube 20 gradually increases from its nominal diameter d₁ to its maximum diameter d₂. For example, in the illustrated embodiment, the proximal region 50 extends from a point distal to the proximal end 20p of the elongate hollow tube 20 to the proximal-most end 20p of the elongate hollow tube 20. In some embodiments, the point at which the diameter of the elongate hollow tube 20 begins to increase can be at or proximal to a sealing element disposed within the elongate hollow tube 20. For example, the tapered portion of the elongate hollow tube 20 in the proximal region 50 can extend distally from a maximum diameter at the proximal end 20p to a nominal diameter at or near a seal 60 disposed within the elongate hollow tube 20. In this manner, the shape of the proximal region 50 can guide an inserted instrument into substantial alignment with a sealing element, e.g., seal 60, as the instrument is inserted. Guiding an instrument into alignment with a seal can, for example, reduce potential damage to the seal. In other embodiments, the length of the proximal tapering region 50 can be expressed as a percentage of the overall length of the elongate hollow tube 20. For example, the proximal region 50 can extend distally from the proximal end 20p of the elongate hollow tube 20 for a distance of approximately 20% of the overall length of the elongate hollow tube 20. In other embodiments, the proximal region 50 can extend distally from the proximal end 20p of the elongate hollow tube 20 for a distance between approximately 20% and 40% of the overall length of the elongate hollow tube 20, such as for example, one third (33%) of the overall length of the elongate hollow tube 20.

The distal region 40 of the elongate hollow tube 20 can have any shape, but as shown it has a cylindrical shape with a constant diameter. The distal end 20d can also have any shape. In some embodiments, the distal end 20d can include an angled portion to enable the cannula 10 to be inserted through an opening formed in tissue to position the distal region 40 of the cannula 10 within a body cavity. For example, the distal-most end 20d can have an angled surface that can include a cutting edge 80. The cutting edge 80 can create an incision as the surgical access device is inserted into tissue.

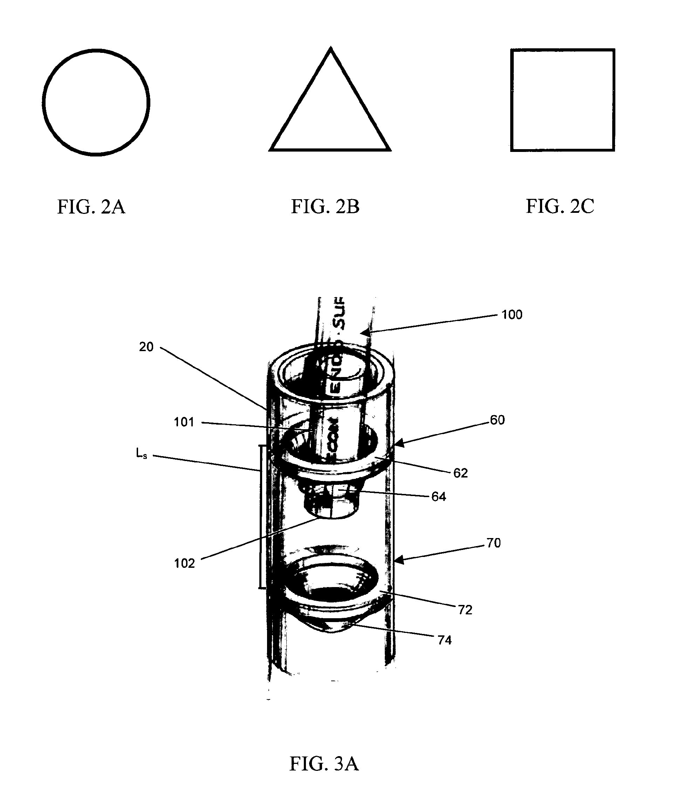

The elongate hollow tube 20 can also have any cross-sectional shape. For example, the elongate hollow tube 20 can have a substantially circular cross-section, as shown in FIG. 1 and FIG. 2A. In other embodiments, the elongate hollow tube 20 can have a non-circular cross-section. For example, the non-circular cross-section of the elongate hollow tube 20 can be a polygon. The cross-section can be a regular polygon such as a triangle, as shown in FIG. 2B, or a square, as shown in FIG. 2C. Non-circular cross-sections, as shown in the exemplary embodiments of FIGS. 2B and 2C, can provide enhanced stiffness in comparison with a circular cross-section (FIG. 2A) of an equivalent diameter as well as other advantages discussed in more detail below.

Tubes with non-circular cross-sections can also provide an advantage by allowing larger instruments to pass through an equivalent sized incision because the maximum internal and external dimensions of the non-circular tube are greater than the equivalent dimensions for a circular tube of the same circumference. For purposes of the following examples, the maximum internal and external dimension of a tube are defined as the maximum dimension of a cross-section taken perpendicular to the longitudinal axis of the tube. For example, a tube with a circular cross-section has a maximum internal dimension determined by the internal circumference divided by π, which represents the internal diameter of a circular tube. In contrast, a tube with a triangular cross-section has a maximum internal dimension, i.e., the length of one of the internal sides of a triangular tube, determined by the internal circumference divided by three. Three is less than π, so the maximum internal dimension of a triangular tube will be greater than the maximum internal dimension of a circular tube of the same circumference. As a more specific example, a triangular tube with a maximum dimension of 2 mm, i.e., the length of one of the sides of the triangular tube, would have a circumference of 6 mm. In comparison, a circular tube with an overall circumference of 6 mm has a maximum dimension, i.e., diameter, of only about 1.9 mm. Thus for the same circumference, a triangular tube provides a greater internal dimension. A greater internal dimension allows larger instruments to pass through the cannula for a given incision size because the circumference of the cannula determines the incision size through which the cannula can be inserted. For example, a linear incision in tissue that is 3 mm long can accommodate a cannula with an external circumference of 6 mm. However, the maximum dimension of a triangular cannula with a circumference of 6 mm is greater than the maximum dimension of a circular cannula with a circumference of 6 mm, thus allowing larger instruments to be inserted through the triangular cannula.

Typically, during surgical procedures in a body cavity, such as the abdomen, insufflation is provided to expand the body cavity to facilitate the surgical procedure. Thus, in order to maintain insufflation within the body cavity, most surgical access devices include at least one seal disposed therein to prevent air and/or gas from escaping when surgical instruments are inserted therethrough. The at least one seal that forms the sealing element can have a variety of sizes, shapes, and configurations. Various sealing elements are known in the art and can be used, including an instrument seal that forms a seal around an instrument disposed therethrough, but otherwise does not form a seal when no instrument is disposed therethrough; a channel seal or zero-closure seal that seals the working channel created by the sealing port when no instrument is disposed therethrough; or a combination instrument seal and channel seal that is effective to both form a seal around an instrument disposed therethrough and to form a seal in the working channel when no instrument is disposed therethrough. A person skilled in the art will appreciate that any instrument and/or channel seal can be used. Exemplary sealing elements include, for example, duckbill seals, flapper valves, gel seals, diaphragm seals, lip seals, iris seals, non-linear sealing elements such sealing elements with an S-shaped opening, cone seals, multi-layer seals, septum valves, or any other seal known in the art can be used. A person skilled in the art will also appreciate that any number of seals and any combination of seals can be included at any location in any of the embodiments described herein, whether or not the seal combinations are specifically discussed in the corresponding description of a particular embodiment. Exemplary seal configurations are described in more detail in U.S. Publication No. 2004/0230161 entitled "Trocar Seal Assembly," filed on March 31, 2004, and U.S. Application Serial No. 10/687,502 entitled "Conical Trocar Seal," filed on October 15, 2003, which are hereby incorporated by reference in their entirety.

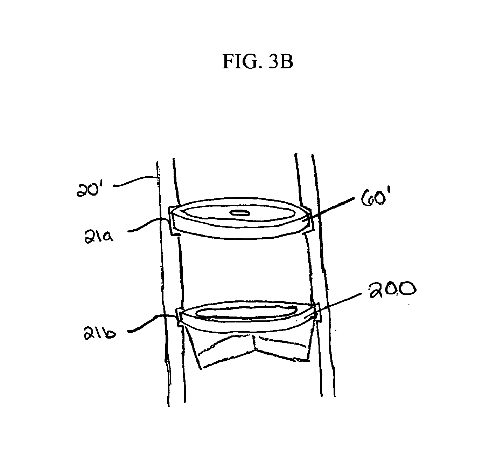

In an exemplary embodiment shown in FIGS. 1 and 3, a sealing element including a first seal 60 and a second seal 70 can be disposed within the elongate hollow tube 20, however the device can include any number of sealing elements. The first seal 60 and second seal 70 can be spaced apart from each other by a distance Lₛ (FIG. 3) as well as from the proximal and distal ends 20p, 20d of the elongate hollow tube 20. For example, the first seal 60 and second seal 70 can be spaced apart from the proximal end 20p of the elongate hollow tube 20 by a distance L₁. Spacing the seals 60 and 70 apart from the proximal end 20p of the elongate hollow tube 20 can, for example, allow a protective element, such as those discussed in more detail below, to be included proximal to the seals 60, 70. For example, the first seal 60 can be disposed between about 1 mm and 10 mm from the proximal end 20p of the elongate hollow tube 20. In another embodiment, L₁ can be approximately equal to the typical thickness of the tissue wall through which the device will be inserted, i.e., the distance between the outer surface 90 and the inner surface 91 of the tissue wall. Depending upon the length of the cannula 10, the first and second seals 60, 70 can also be spaced apart from the distal end 20d of the cannula by a distance L₂.

The first and second seals 60, 70 can be spaced apart from each other at any distance. For example, FIG. 3 illustrates the first and second seals 60, 70 that form the sealing element in more detail. A person skilled in the art will appreciate that, while two seals 60, 70 are shown, the device can include any number of seals at any location. In the illustrated embodiment, an instrument 100 can be inserted through the first seal 60 and then through the second seal 70. As illustrated in FIG. 3, the first and second seals 60, 70 can be spaced apart by a distance Lₛ. The distance Lₛ can vary and is generally determined by the type of valve and the amount of space required to allow an instrument 100 to pass through the seals 60, 70. In some embodiments, the distance Lₛ can be approximately equal to the length of the elongate hollow tube 20, or a percentage of the length of the elongate hollow tube such as, for example, between about 10% and 90% of the length. For example, the first seal 60 can be disposed between about 1 mm and 10 mm from the proximal end 20p of the elongate hollow tube 20 and the second seal 70 can be disposed within the elongate hollow tube 20 at or near the distal end 20d of the elongate hollow tube 20. For example, the second seal 70 can be disposed proximal to the opening formed in the distal end 20d. In other embodiments, the distance Lₛ can be minimized so as to maximize the range of motion of an inserted instrument 100 within the elongate hollow tube 20.

In another embodiment, the sealing element can be disposed within the elongate hollow tube 20 at a position effective to maintain contact between the sealing element and an instrument inserted therethrough as the instrument is rotated or pivoted. This position, i.e., the pivot point or point about which an instrument is pivoted, can correspond to the neutral point of an inserted instrument. The neutral point of an instrument is the point that will remain stationary or neutral with respect to the cannula when the instrument is rotated or pivoted. Positioning the sealing element at the neutral point of an instrument can be effective to reduce any radial displacement of the instrument with respect to the center of the elongate hollow tube 20 during manipulation of the instrument. Such a configuration can also reduce the number of points constraining motion of the instrument and cannula system. For example, when the cannula and an inserted instrument pivot about approximately the same point, the number of points constraining motion of the system can be reduced to one.

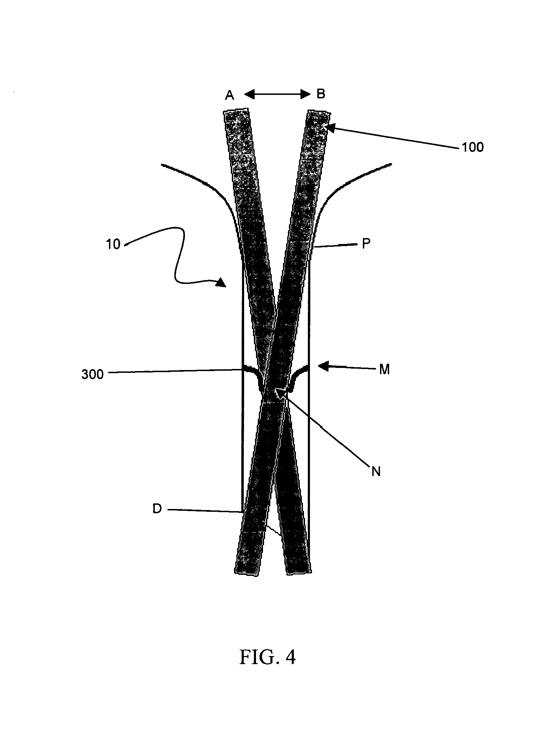

The neutral point can generally be determined by finding a midpoint or mid-range between the proximal and distal contact points of the elongate hollow tube 20 and an instrument disposed therethrough when the instrument is angled within the cannula. This is illustrated for example in FIG. 4 which schematically shows a surgical access device 10 including a sealing element 300 and an inserted instrument 100 at two positions A and B. As illustrated, the instrument pivots about a point N as it is moved from a first position A to a second position B within the access device 10. As the instrument 100 is moved within the device, it can contact the surgical access device 10 at a distal contact point D and a proximal contact point P and can pivot about a point N that represents the neutral point of the instrument. As discussed above, the sealing element 300 can be positioned at the neutral point N of instrument 100. In the illustrated embodiment, for example, the sealing element is positioned at approximately the midpoint M between the proximal contact point P and the distal contact point D. This approximate midpoint M between the proximal contact point P and the corresponds to the neutral point N of the inserted instrument 100, i.e., the point that will remain stationary or neutral with respect to the surgical access device 10 as the instrument 100 is rotated or pivoted, for example between position A and position B.

Because the neutral point may vary between instruments, i.e., the proximal and distal contact points P, D may vary with the size and shape of the instrument 100, the sealing element 300 need not be positioned at a specific midpoint M for a particular instrument. Instead, a mid-range can be determined for a number of instruments, and the seal assembly can be positioned within that mid-range. The neutral point may also vary if the surgical access device has an angled proximal end. In particular, the distance between the proximal and distal contact points (i.e., the contact length) may vary as an instrument is rotated in a radial fashion around a surgical access device having an angled proximal end. In such a configuration, the neutral point can be determined by finding a nominal midpoint that is positioned between the midpoints that correspond to the varying contact lengths.

The sealing element(s) can also be held in the elongate hollow tube using various techniques. In one embodiment, as shown in FIG. 3A, each seal 60, 70 generally includes a peripheral rim or flange 62, 72 and a seal segment 64, 74. The flange 62, 72 can anchor the seal within the elongate hollow tube and the seal segment 64, 74 can provide a seal around an inserted instrument 100. As discussed above, the seals can be any type of seal and can be disposed in any quantity and order within the elongate hollow tube. In the illustrated embodiment, the first and second seals 60, 70 are both instrument seals that form a seal around an instrument 100 disposed therethrough, but otherwise do not form a seal when no instrument is disposed therethrough. In this embodiment, only non-zero closures seals are present. No channel seal is present. In other embodiments, one of the seals, or an additional seal, can be a channel seal or zero-closure seal that seals the working channel in the elongate hollow tube 20 when no instrument is disposed therethrough. For example, the first seal can be an instrument seal and the second seal can be a channel seal or zero-closure seal. This is illustrated in FIG. 3B, which shows a portion of another embodiment of a hollow tube 20' having a proximal sealing element in the form of a lip seal 60', i.e., an elastomeric seal with a central opening that forms a seal around an instrument. The lip seal 60' be formed from a unitary molded composite, or it can include a flexible sealing member having a flexible or rigid support housing disposed therearound for mating the lip seal 60' to the hollow tube 20', as will be discussed in more detail below. As further shown in FIG. 3B, the hollow tube 20' also includes a distal sealing element in the form of a duck-bill seal 200 that seals the channel of the tube when no instrument is inserted therethrough. When the seals are positioned within the tube, they will generally extend across the longitudinal axis of the tube, e.g., in a transverse or perpendicular orientation to the axis.

As discussed above, the elongate hollow tube 20 can have a non-circular cross-section. The sealing elements can thus be shaped to correspond to the non-circular cross-section of the elongate hollow tube. For example, the seals can have a non-circular shaped opening to receive a surgical instrument having a non-circular cross-section.

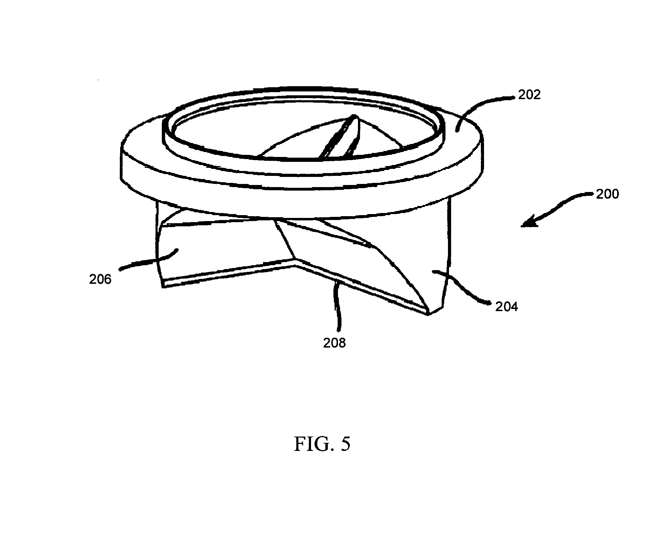

As noted above, one type of sealing element that can be used in the surgical access device is the channel or zero-closure seal, an example of which is shown in more detail in FIG. 5. As shown, the illustrated zero-closure seal is in the form of a duckbill seal 200. The seal 200 is configured to form a seal in a working channel when no instrument is disposed therethrough to thus prevent the leakage of insufflation gases delivered through the surgical access device to the body cavity. As shown, the duckbill seal 200 can have a generally circular flange 202 with a sidewall 204 extending distally therefrom. The shape of the sidewall 204 can vary, but in the illustrated embodiment, the sidewall 204 includes opposed flaps 206 that extend at an angle toward one another in a distal direction and that come together at a distal end to form a seal face 208. In other embodiments, the opposed flaps 206 can extend toward one another with no angle to form a seal face 208 that is parallel relative to the circular flange 202. The opposed flaps 206 can be movable relative to one another to allow the seal face 208 to move between a closed position, in which no instrument is disposed therethrough and the seal face 208 seals the working channel of the surgical access device, and an open position in which an instrument is disposed therethrough. The seal can include various other features, as described in more detail in U.S. Application No. 11/771,263, entitled "Duckbill Seal with Fluid Drainage Feature," filed on June 29, 2007, which is hereby incorporated by reference in its entirety. In addition, the seal face 208 of the seal 200 can be in any nonlinear shape or configuration known in the art, for example in an S-shaped configuration, as described in more detail in U.S. Patent No. 5,330,437, entitled "Self Sealing Flexible Elastomeric Valve and Trocar Assembly for Incorporating Same," filed November 12, 1993, which is hereby incorporated by reference in its entirety.

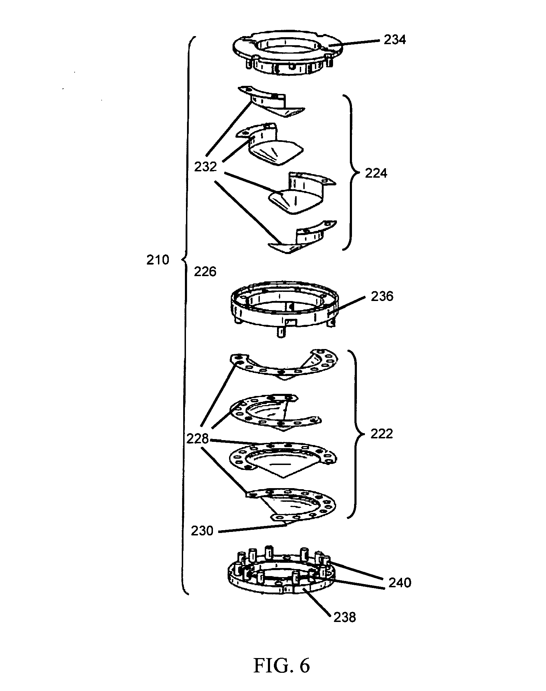

As noted above, another type of sealing element that can be used in the surgical access device is an instrument seal, an example of which is shown in more detail in FIG. 6. As shown, the instrument seal 218 is generally in the form of a multi-layer protective member 224 disposed on a proximal surface 226 of a multi-layer conical seal 222. The multi-layer conical seal 222 can include a series of overlapping seal segments 228 that are assembled in a woven arrangement to provide a complete seal body. The seal segments 228 can be stacked on top of one another or woven together in an overlapping fashion to form the multi-layer seal 222 having a central opening 230 therein. The seal segments 228 can be made from any number of materials known to those skilled in the art, but in an exemplary embodiment the seal segments 228 are formed from an elastomeric material. The seal segments 228 can also be molded such that they have a varying thickness across the profile of the seal 222. Varying the thickness across the profile of the seal 222 can be effective to minimize leakage and reduce drag forces on instruments passed therethrough. The multi-layer protective member 224 can similarly be formed from a series of overlapping segments 232 that are disposed proximal to the overlapping seal segments 228 and that are configured to protect the seal segments 228 from damage caused by surgical instruments inserted through the opening 230 in the seal 222. The protective member 224 can also be formed from various materials, but in certain exemplary embodiments the protective member 224 is formed from a molded thermoplastic elastomer. The segments 228, 232 that form the seal 222 and the protective member 224 can be held together using various techniques known in the art. As shown in FIG. 6, the segments 228, 232 are held together by several ring members that mate to engage the segments 228, 232 therebetween. In particular, the protective member 224 is engaged between a crown 234 and a gasket ring 236, and the seal 222 is engaged between the gasket ring 236 and a retainer ring 238. Pins 240 are used to mate the ring members 234, 236, 238 and to extend through and engage the segments of the seal 222 and the protective member 224.

Several techniques can be used to incorporate a sealing element into the elongate hollow tube 20 of the surgical access device 10. For example, the sealing element, e.g., seals 60, 70, can be mated or affixed to an inner wall of the cannula as shown in FIGS. 1 and 3A. One of ordinary skill in the art will appreciate that the seals 60, 70 can be mated or affixed to an inner wall of the elongate hollow tube 20 in any number of ways. For example, an outer rim of a seal can be joined to the inner surface of the elongate hollow tube 20 utilizing techniques such as sonic welding and adhesives. Alternatively, the sealing element can be integrally formed with the elongate hollow tube 20 such that the sealing element forms at least a portion of a sidewall of the elongate hollow tube 20. In another embodiment, the sealing element and the elongate hollow tube 20 can be manufactured simultaneously to form one unitary structure. In yet another embodiment, the hollow tube 20' can include one or more radially grooves formed on an inner wall thereof that can be configured to seat an peripheral rim or flange on a sealing element. For example, FIG. 3B illustrates a proximal groove 21a that seats the rim of the lip seal 60', and a distal groove 21b that seats the rim of the duckbill seal 200. While grooves are shown, the inner tube can alternatively include one or more stepped regions. Such a configuration can allow the sealing element to simply be advanced down into the tube to be seated at a desired location.

When fully assembled, one or more instrument seals 218 can be disposed at various locations within the elongate hollow tube 20 of the surgical access device 10, as discussed above. In use, an instrument can be inserted into the center of the seal assembly and the seal segments 228, 232 can engage and form a seal around an outer surface of the instrument to thereby prevent the passage of fluids through the seal 218. When no instrument is inserted therethrough, the opening will not form a seal, however other configurations in which a seal is formed when no instrument is inserted therethrough are also conceivable.

In use, exemplary surgical access devices provide for greater maneuverability of surgical instruments within a patient while maintaining insufflation. In one embodiment, this greater maneuverability can be provided by minimizing the number of points at which an instrument inserted through the surgical access device is constrained by contact with elements of the surgical access device. The surgical access device itself is constrained by contact between the device and the walls of the incision through which the device is inserted. An instrument inserted through the device is further constrained by contact with the device, particularly at the proximal and distal ends and at any seal locations. In an exemplary embodiment such as illustrated in FIG. 1, the number of points constraining the movement of an inserted instrument can be minimized by configuring the cannula 10 such that the sealing elements 60, 70 are disposed within the elongate hollow tube 20 at approximately the same location where movement of the elongate hollow tube 20 is also constrained by the inner surface 91 of a tissue wall. As discussed above, the maneuverability of the device can also be improved by reducing the distance (Lₛ in FIG. 3A) between the sealing elements 60, 70. In other embodiments, the maneuverability of surgical instruments can be improved by providing a cannula 10 in which one or more portions are substantially flexible. For example, the access device, or portions thereof, can be formed of any suitable material known in the art, for example silicone, urethane, thermoplastic elastomer, and rubber.

The present invention also provides methods for accessing a body cavity. In one exemplary embodiment, an opening can be formed in tissue, for example, in the abdominal wall or umbilicus, and the distal region 40 of the elongate hollow tube 20 of the cannula 10 can be inserted through an opening formed in tissue to position the distal region 40 of the cannula 10 within a body cavity. In another embodiment, in which the distal region 40 includes an angled surface that can include a cutting edge 80, the cutting edge 80 can create an incision as the surgical access device is inserted into tissue. In one exemplary embodiment, a proximal-most portion 30 of the cannula 10 can rest flush or substantially flush against a tissue surface 200 such that no portion of the cannula extends above the tissue surface, other than maybe a portion of the rim. An instrument 100 can then be inserted through an opening in the proximal end of the cannula and through a working channel of the cannula 10 to position a distal end of the instrument 100 in the body. A sealing element(s) located in the working channel can form a seal around the instrument inserted therethrough. The inserted instrument 100 can then be manipulated within the cannula 10 to perform various surgical procedures within the body cavity.

As discussed in more detail above, the proximal region 50 of the cannula 10 can be shaped so as to guide an inserted instrument 100 into the working channel of the cannula 10. The shape of the proximal region 50 can also guide an inserted instrument into substantial alignment with the central opening of a sealing element, e.g., seal 60, as the instrument is inserted. Guiding an instrument into alignment with a seal can reduce potential damage to the seal, for example.

In some embodiments, inserting the cannula can also include positioning a proximal-most portion of the cannula against a tissue surface and positioning at least one sealing element disposed within the working channel of the cannula 10 within at least one of an opening in the tissue and the body cavity. In other embodiments, a second sealing element can be disposed in the working channel of the cannula 10. The second sealing element can be positioned within at least one of the opening in the tissue and the body cavity.

As discussed above, the cannula 10 can be configured in any number of ways. As discussed above, the working channel of the cannula 10 can be configured to allow an inserted instrument to contact at least one of a distal-most portion and the proximal-most portion of the cannula. In some embodiments, the neutral point of the inserted instrument, discussed in more detail above, can be positioned at the neutral, or pivot point, of the cannula within the tissue. For example, an instrument inserted through the cannula can pivot about a sealing element disposed in the working channel so as to contact at least one of a distal-most portion and the proximal-most portion of the cannula.

As discussed in detail above, the proximal-most portion of the cannula 10 can extend radially outward to engage the tissue surface and to prevent the proximal-most end of the cannula 10 from passing through the opening in the tissue. The shape of the distal surface of the proximal-most end of the cannula 10, i.e., the outer surface, can also be configured to allow the cannula 10 to pivot within the incision while maintaining engagement between the cannula and the tissue surface. For example, when the cannula is positioned such that the at least one sealing element is disposed within an opening in the body cavity, as discussed above, the cannula and an inserted instrument pivoting about the sealing element can pivot about approximately the same point. Such a configuration can reduce the number of points constraining motion of the instrument and cannula system. For example, when the cannula and an inserted instrument pivot about approximately the same point, the number of points constraining motion of the system can be reduced to one.

The radially extending proximal-most portion 50 of the cannula, which is discussed in more detail above, can also provide increased freedom of movement of an inserted instrument within the cannula. For example, when the inserted instrument is pivoted or rotated within the working channel, i.e., the elongate hollow tube 20, of the cannula 10, the instrument can engage the proximal or distal end of the cannula, or both. The larger diameter of the cannula 10 at the proximal-most end 30 can allow an inserted instrument to be angled within the cannula, for example. In an exemplary embodiment in which the cannula is flexible or substantially flexible, an inserted instrument can be angled within the tapered proximal region 50 to engage the distal end 40 of the cannula so as to flex the elongate hollow tube 20, thereby providing increased range of motion of the instrument within the body cavity.

The embodiments described herein can be used in any known and future surgical procedures and methods, as will be appreciated by those skilled in the art.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam, and a liquid bath (e.g., cold soak).

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

What is claimed is:

## Claims

1. A surgical access device, comprising:
a rigid elongate hollow tube having proximal and distal ends, the distal end being configured to extend through a body wall into a body cavity and the proximal end being configured to be positioned adjacent to a tissue surface, and wherein the proximal end includes a rim having a distal surface that contacts a tissue surface and a proximal surface that forms the proximal-most end of the tube and that defines an opening extending into a working channel of the hollow tube; and
a sealing element disposed within the hollow tube and configured to form a seal around an instrument inserted through the sealing element.

2. The surgical access device of claim 1, wherein the rim gradually extends radially outward.

3. The surgical access device of claim 1, wherein the sealing element is spaced a distance apart from the proximal-most end of the elongate hollow tube.

4. The surgical access device of claim 1, wherein the sealing element is spaced a distance apart from the distal-most end of the elongate hollow tube.

5. The surgical access device of claim 1, wherein the sealing element comprises a first sealing element, and wherein the device further comprises a second sealing element disposed within the hollow tube and configured to seal the working channel of the elongate hollow tube when no instrument is disposed through the second sealing element.

6. The surgical access device of claim 1, wherein the sealing element comprises a unitary molded structure held within a groove formed in an inner surface of the elongate tube.

7. The surgical access device of claim 1, wherein the sealing element and the elongate hollow tube are formed from different materials.

8. The surgical access device of claim 1, wherein the elongate hollow tube has a non-circular cross-section.

9. A surgical access device, comprising:
an elongate hollow tube having a gradually radially expanding proximal-most end defining an opening extending into a working channel of the tube, the proximal-most end being configured to rest against a tissue surface when a distal portion of the elongate hollow tube is disposed through an opening in tissue and into a body cavity; and
a sealing element disposed within the hollow tube and configured to form a seal around an instrument inserted through the sealing element.

10. The surgical access device of claim 9, wherein the sealing element is spaced a distance apart from the distal-most end of the elongate hollow tube.

11. The surgical access device of claim 9, wherein the sealing element comprises a first sealing element, and wherein the device further comprises a second sealing element disposed within the hollow tube and configured to seal the working channel of the elongate hollow tube when no instrument is disposed through the second sealing element.

12. The surgical access device of claim 9, wherein the sealing element comprises a unitary molded structure held within a groove formed in an inner surface of the elongate tube.

13. The surgical access device of claim 9, wherein the elongate hollow tube has a non-circular cross-section.

14. The surgical access device of claim 9, wherein the elongate hollow tube is rigid.
